# EUROPEAN PATENT SPECIFICATION

(11) **EP 2 872 162 B1**
(45) Date of publication and mention of the grant of the patent: **06.09.2017**
(21) Application number: 13740048.7
(22) Date of filing: 12.07.2013
(51) Int. Cl.: A61K 38/16, A61K 35/74, A23L 33/135, A61P 1/00, A61P 31/04, C12N 9/22, C07K 14/245

(54) **COLICINS FOR TREATING BACTERIAL INFECTIONS**
COLICINE ZUR BEHANDLUNG VON BAKTERIELLEN INFEKTIONEN
COLICINES POUR LE TRAITEMENT D'INFECTIONS BACTÉRIENNES

(30) Priority: 13.07.2012 GB 201212588
(43) Date of publication of application: 20.05.2015
(73) Proprietor: The University Court of The University Of Glasgow, Glasgow, Strathclyde G12 8QQ (GB)
(72) Inventor: WALKER, Daniel, Glasgow Strathclyde G12 8AT (GB); SMITH, Karen, Kilmarnock Ayrshire KA2 0BE (GB)
(74) Representative: Forrest, Graham Robert
(86) International application number: PCT/GB2013/051858
(87) International publication number: WO 2014/009744

(56) References cited:
- US-A1- 2008 233 086
- CARLA L. BROWN ET AL: "Colicin-like bacteriocins as novel therapeutic agents for the treatment of chronic biofilm-mediated infection", BIOCHEMICAL SOCIETY TRANSACTIONS, vol. 7, no. 6, 21 November 2012 (2012-11-21), pages 521-1552, XP055079887, ISSN: 0300-5127, DOI: 10.3748/wjg.v17.i14.1797
- MARUSKA BUDIC ET AL: "Escherichia coli Bacteriocins: Antimicrobial Efficacy and Prevalence among Isolates from Patients with Bacteraemia", PLOS ONE, vol. 6, no. 12, 19 December 2011 (2011-12-19), page e28769, XP055079354, ISSN: 1932-6203, DOI: 10.1371/journal.pone.0028769
- A. BARNEOUD-ARNOULET ET AL: "Toxicity of the Colicin M Catalytic Domain Exported to the Periplasm Is FkpA Independent", JOURNAL OF BACTERIOLOGY, vol. 192, no. 19, 1 October 2010 (2010-10-01), pages 5212-5219, XP055079905, ISSN: 0021-9193, DOI: 10.1128/JB.00431-10
- I. INGRASSIA ET AL: "Lactobacillus casei DN-114 001 Inhibits the Ability of Adherent-Invasive Escherichia coli Isolated from Crohn's Disease Patients To Adhere to and To Invade Intestinal Epithelial Cells", APPLIED AND ENVIRONMENTAL MICROBIOLOGY, vol. 71, no. 6, 1 June 2005 (2005-06-01), pages 2880-2887, XP055079653, ISSN: 0099-2240, DOI: 10.1128/AEM.71.6.2880-2887.2005

## Description

### Field of Invention

The present invention relates to compositions for use in the treatment of Crohn's disease, or the treatment of an adherent-invasive Escherichia coli infection.

### Background to the invention

Colicins are high molecular mass, typically plasmid-encoded protein toxins that target specific strains of *E. coli.* They are produced by approximately 30% of natural *E. coli* isolates. Their primary function is thought to be to reduce competition from related bacteria during times of environmental stress. Colicins are well characterised and their cytotoxic activities and mechanisms of entry into target cells are understood in molecular detail (Loftus et al 2006; Walker et al, 2007). Colicins may kill susceptible cells through membrane depolarisation (for example, colicins A, B, N, IA and E1), a non-specific DNase activity (for example, colicins E2, E7, E8 and E9), a highly specific RNase activity directed against ribosomal RNA (colicins E3, E4 and E6) or tRNA (colicins E5 and D), or in the case of colicin M by the inhibition of cell wall synthesis. Colicins are typically able to kill susceptible cells at nM concentrations since they bind with high affinity to their specific outer membrane receptor. For example, colicin E9 has been shown to bind to its outer membrane receptor BtuB with an affinity of 2 nM (Housden et al., 2005).

Crohn's disease (CD) is a chronic, debilitating and currently incurable form of inflammatory bcwel disease of unknown cause affecting around 1:1000 people in the UK. A higher incidence of 1:300 in the US has been reported. The development of CD is multi-factorial, but one area of research interest is the abnormal colonisation of the ileal mucosa by pathogenic adherent-invasive *E. coli* (AIEC). These bacteria colonise the surface of the ileal mucosa of Crohn's patients as a thick biofilm and have the ability to invade and replicate within epithelial cells (Boudeau et al 1999). Animal models of Crohn's disease provide further evidence for a pivotal role of AIEC in this condition. AIEC but not non-pathogenic *E. coli* K-12 strains were shown to aggravate colitis in an injured mouse colon model of colitis (Carvalho et al, 2008). Additionally, transgenic mice expressing human CEACAM6, which is required as a receptor for the adhesion of AIEC, are susceptible to colonisation by AIEC and consequently develop severe colitis whereas colonisation and the development of colitis is absent in wild-type mice (Carvalho et al, 2009). The recently published genome sequence of the reference strain LF82 indicates AIEC possesses a range of known virulence factors (Miquel et al, 2010). The capacity to bind and invade epithelial cells (Boudeau et al, 1999) and to form biofilms are known phenotypic features of AIEC (Martinez-Medina et al, 2009). AIEC are also capable of invading and replicating within macrophages. Infection of host cells by AIEC leads to the overproduction of key proinflammatory cytokines such as TNF-α, which may contribute to the inflammation and disease pathogenesis in Crohn's disease (Glasser et al, (2001)).

As in other chronic bacterial infections, the ability to form biofilms is expected to play a key role in the persistence of AIEC in the gut and their resistance to antibiotics. Biofilm formation is known to be a key factor in the pathogenesis of a range of chronic bacterial infections such as *Pseudomonas aeruginosa* lung infections in cystic fibrosis patients and recurrent *E. coli* urinary tract infections. Relative to the planktonic state, biofilms, which are essentially surface attached bacterial communities surrounded by an extracellular polymeric matrix, can show a 10 to 1000 fold increase in their resistance to antibiotics and also show increased resistance to host defences. The activity of protein and peptide antibiotics against bacteria in biofilms is not well understood. Production and secretion of proteases is a known phenotype of some bacterial biofilms. Proteases will readily inactivate protein antibiotics. The extracellular matrix may also act as a diffusion barrier to large antibiotics, thus reducing their efficiency.

Currently CD is not well treated. There is no cure for CD and treatment is focused on alleviating symptoms using, for example, corticosteroids, aminosalicylates or immunosuppressants. Injectable anti-TNF-alpha antibodies and antibody fragments, have been developed for the treatment of IBD and are effective in a subpopulation of CD patients. However, these treatments are expensive and not without significant side effects.

Limited studies have shown some clinical benefit in use of antibiotics in the management of Crohn's disease although there is relatively weak evidence for their efficacy (Prantera and Lia Scribano, 2009). The most commonly used antibiotics for the management of Crohn's disease are metronidazole and the broad spectrum fluoroquinolone antibiotic, ciprofloxacin. However, the long term use of broad spectrum antibiotics is associated with serious complications including *Clostridium difficile* infection.

An alternative approach to the treatment of Crohn's disease is the use of probiotics, which are living microbial food ingredient with a beneficial effect on human health. However, a systematic review of trial data failed to show any benefit from the use of a range of probiotics in the treatment of Crohn's (Rolfe et al, 2006).

Thus, there remains in the art a need for alternative treatments for CD.

### Summary of the invention

The present inventors have discovered that colicins are highly active against AIEC isolates obtained from patients with Crohn's disease. The colicins are effective not only against the free bacteria, but also against AIEC grown in the biofilm state. This is surprising because bacteria in a biofilm are usually highly resistant to antibiotics and the colicins are high molecular weight proteins that would not be expected to efficiently penetrate the extracellular matrix of a biofilm. In some cases, the colicins were more effective against AIEC in a biofilm than antibiotics that are known to have some effect in the treatment of Crohn's disease. AIEC grown in a biofilm were also killed by the addition of bacteria that were engineered to produce a colicin. Thus, colicin producing bacteria could be used to deliver colicins to AIEC growing in the body. Even more surprisingly, the inventors discovered that colicins kill intracellular AIEC grown in infected macrophages. Without wishing to be bound by theory, this suggests that colicins taken up in macrophage vacuoles are not inactivated in the intracellular environment and that vacuoles containing the colicin fuse with vacuoles containing AIEC, neither of which could have been predicted. Colicins therefore have characteristics that make them useful in therapeutic methods of treatment for intracellular or biofilm associated bacterial infections, for example in patients with Crohn's disease.

In some cases, the colicin may be directly administered to a patient in need thereof. Thus, in some aspects, the invention provides a colicin for use in a method of treating Crohn's disease or treating an AIEC infection, including when the AIEC infection is associated with a biofilm and/or is an intracellular bacterial infection.

In another aspect, the invention provides a polynucleotide that encodes a colicin for use in a method of treating Crohn's disease or treating an AIEC infection, including when the AIEC infection is associated with a biofilm and/or is an intracellular bacterial infection.

AIEC designates a pathogenic group of E. coli. LF82 is the prototype strain. In some cases in accordance with the invention, an E. coli bacterium is an AIEC bacterium if it meets the following criteria: (i) ability to adhere to and to invade intestinal epithelial cells, (ii) ability to survive and to replicate extensively in large vacuoles within macrophages without triggering host cell death, and (iii) ability to induce the release of TNF-α by infected macrophages. In other cases, AIEC bacteria can be identified by phylogenetics, as described in Miquel et al (2010), which reports that AIEC fall within a distinct AIEC clonal phylogenetic complex of E. coli. It is thought that AIEC may also be the cause of some extraintestinal infections (Martinez-Medina et al. (2009)).

In some cases in accordance with the invention, the AIEC infection may be associated with a biofilm. A bacterial infection is "associated with a biofilm" when the bacterial cells of the infection are surface attached and embedded in a matrix generally composed of bacterially secreted polymers.

AIEC also invade and replicate within patient epithelial cells and macrophages. The infected cells overproduce TNF-α and other pro-inflammatory cytokines, which may contribute to inflammation and disease pathogenesis. Thus, in some cases in accordance with the invention, the treatment targets an intracellular AIEC infection. The bacteria may be in epithelial cells and/or in macrophages. Preferably, the treatment targets AIEC in macrophages.

In other cases, the treatment targets both bacteria that are associated with a biofilm, such as AIEC, and intracellular bacteria. A treatment that targets both the biofilm and intracellular bacteria may be more effective because fewer bacterial cells will remain after treatment that could reestablish the infection.

In some cases, the colicin can be delivered in the form of a bacterium that produces one or more colicins. Thus, in some aspects, the invention provides a bacterium for use in a method of treating Crohn's disease or treating an AIEC infection, including when the AIEC infection is associated with a biofilm and/or is an intracellular bacterial infection, wherein the bacterium produces a colicin.

The bacterium may be any bacterium that is suitable for administering to a subject. The bacterium may naturally produce a colicin (i.e. an endogenous colicin) or the bacterium may have been genetically engineered to produce a colicin (i.e. a heterologous collicin). It will be understood that the bacterium may also have been engineered to enhance production of an endogenous colicin which is already naturally produced by the bacterium. Preferably the strain is non-pathogenic. Lactobacilli, Bifidobacteria, Lactococci and non-pathogenic strains of E. coli are examples of suitable bacteria. For example, the bacteria could be E. coli K-12 W3110 carrying a plasmid that encodes a colicin, for example colicin E1 plasmid pColE1-K53.

The bacterium is capable of producing the colicin at the site of the infection, for example in the gut of a subject that has Crohn's disease. The bacterium releases the colicin, for example by secretion or following expression of a colicin release protein. In some cases in accordance with the invention, the production and/or release of the colicin is induced under the conditions encountered during administration of the bacterium or the conditions encountered at the site of the infection. For example, production and/or release of the colicin may be induced under the conditions found in the digestive system or in the ileum of the patient. Expression of the colicin may be under the control of a promoter that is activated under suitable conditions, for example, when the bacterium is in competition with other bacteria in the gut. The promoter may be an SOS-responsive promoter. An example promoter is the LexA promoter.

In some cases, the bacterium may be in a form in which the activity of the bacterium is suspended. For example, the bacterium may be freeze-dried. Production and/or release of the colicin is resumed during or after administration of the bacterium to the subject or when the bacterium reaches the site of the infection. The bacterium may produce more than one colicin. For example, the bacterium may produce and release two, three, four or more colicins.

In some cases, bacteria that have a therapeutic effect can conveniently be administered in a food product that has the bacteria as an ingredient. Thus, in some cases in accordance with the invention, the bacterium is suitable for use as an ingredient in a food product. Lactococcus lactis, which is used in the production of cheese, is an example of a bacteria that is suitable for use as an ingredient in a food product.

In one aspect, the invention provides a food product for use in a method of treating Crohn's disease or treating an AIEC infection, including when the AIEC infection is associated with a biofilm and/or is an intracellular bacterial infection, wherein the food product comprises a colicin or a bacterium that produces and releases a colicin. The colicin has a therapeutic effect.

A food product is any substance that is suitable for consumption by a subject to provide nutrients, including products consumed in the form of a beverage. The food product may be fortified by the addition of the bacterium as an ingredient. Alternatively, the food product may be a fermented food product fermented by the bacterium or having a fermented ingredient. After consumption, the bacterium in the food product begins or continues to produce the colicin and releases it directly into the gut. Examples of food products that may be used in accordance with the invention are cheese, milk, yogurt and cereals.

In another aspect, the invention provides the use of a colicin, a bacterium that produces a colicin, or a polynucleotide that encodes a colicin in the manufacture of a medicament for treating Crohn's disease or treating an AIEC infection, including when the AIEC infection is associated with a biofilm, or is an intracellular infection.

In another aspect, the invention provides a pharmaceutical composition for use in a method of treating Crohn's disease or treating an AIEC infection, including when the AIEC infection is associated with a biofilm and/or is an intracellular bacterial infection, the composition comprising a pharmaceutically effective amount of a colicin or a bacterium that produces a colicin, and a pharmaceutically acceptable carrier.

Preferably, the pharmaceutical composition is formulated for oral administration. The pharmaceutical composition may be formulated as a food product.

In some cases in accordance with the invention, the pharmaceutical composition is used in a prophylactic method of preventing Crohn's disease in a patient who is at risk of developing the disease, for example a patient having a genetic predisposition to Crohn's disease. Barrett et al (2008) describes example genetic risk factors for Crohn's disease.

Colicins are large protein toxins, having an average 500-600 residues and a molecular weight of around 40 to 80 kDa. Their structure is organised into three domains, each involved in a different stage of their action on a sensitive target cell. The receptor-binding domain (R-domain) is involved in the recognition of a specific receptor on the surface of the target cell. The translocation domain (T-domain) is involved in translocation of the colicin across the target cell outer membrane. The cytotoxic domain (C-domain) has the activity that is toxic to the target cell. Each domain generally functions independently. Thus, the three domains of different colicins are generally interchangeable.

The colicins bind to specific receptors, typically nutrient receptors, on the surface of sensitive cells. Some specific receptors are recognised by several different colicins. For example, colicins E1 to E9 and A all bind to the E. coli vitamin B₁₂ receptor BtuB and colicins Ia and Ib both bind to the Cir receptor. The colicins in accordance with the invention may bind to any suitable receptor on the surface of a target cell. Example receptors are BtuB, FepA, Cir, Tsx, FhuA, OmpF, OmpA, OmpW and IutA. Preferably the colicin binds to FepA, BtuB or Cir. The colicin may be colicin E1, E3, E9, D or Ia. More preferably, the colicin binds to the BtuB receptor. The colicin may be colicin E1 or E9.

Colicins translocate across the outer membrane of a target cell using either the Tol or TonB systems of the target cell. Colicins that use the Tol system are classified as Group A colicins and include colicins A, E1 to E9, K, L, N, S4, U and Y. Colicins that use the TonB system are classified as Group B colicins and include colicins B, D, H, Ia, Ib, M, 5 and 10. The colicins in accordance with the invention may be Group A or Group B colicins. Preferably the colicins are Group A colicins.

The cytotoxic domain of a colicin is typically ionophoric or enzymatic. Ionophoric colicins are pore-forming and kill target cells by depolarisation of the cytoplasmic membrane. Enzymatic colicins typically act as nucleases in the cytoplasm. Colicin M enzymatically degrades components that are needed for cell wall synthesis.

In some cases in accordance with the invention, the colicin is an ionophoric, pore-forming or membrane depolarising colicin. For example, the colicin may be colicin A, B, N, IA, E1, K, U, IB, 5, 10, S4 or Y. Preferably the ionophoric colicin is colicin E1 or colicin IA, more preferably colicin E1.

In other cases in accordance with the invention, the colicin is an enzymatic colicin. For example, the colicin may be a nuclease colicin such as E2, E3, E4, E5, E6, E7, E8, E9, D or the colicin may be colicin M. The colicin may be a DNase. For example, the colicin may be colicin E2, E7, E8 or E9. The colicin may be an rRNase. For example the colicin may be colicin E3, E4 or E6. The colicin may be a tRNase. For example, the colicin may be colicins E5 or D. Preferably the nuclease colicin is colicin E3, E9 or D, more preferably colicin E9.

The outer membrane receptors, translocation pathways and cytotoxic activities of well characterised colicins are shown in Table 1.

**Table 1 - The outer membrane receptors, translocation pathways and cytotoxic activities of well characterised colicins**

| **Colicin** | **Receptor** | **Translocation pathway group** | **Cytotoxic domain** |
|---|---|---|---|
| A, E1 | BtuB | A | Pore-forming |
| E2, E7, E8, E9 | BtuB | A | DNase |
| E3, E4, E6 | BtuB | A | rRNase |
| E5 | BtuB | A | tRNase |
| K | Tsx | A | Pore-forming |
| N | OmpF | A | Pore-forming |
| U | OmpA | A | Pore-forming |
| B | FepA | B | Pore-forming |
| D | FepA | B | tRNase |
| Ia, Ib | Cir | B | Pore-forming |
| 5, 10 | Tsx | B | Pore-forming |
| M | FhuA | B | Inhibits cell wall synthesis |
| S4 | OmpW | A | Pore-forming |
| Y | | A | Pore-forming |

In some cases in accordance with the invention, the colicin comprises a cytotoxic domain that has at least 50%, more preferably 55%, 60%, 65%, 70%, 75%, 80%, 85%, 90%, 95%, 96%, 97%, 98% or 99% amino acid sequence identity to a sequence selected from residues 344-522 of SEQ ID NO: 1 (the colicin E1 cytotoxic domain), residues 451-551 of SEQ ID NO. 2 (the colicin E3 cytotoxic domain), residues 451-582 of SEQ ID NO: 3 (the colicin E9 cytotoxic domain), residues 591-697 of SEQ ID NO: 4 (the colicin D cytotoxic domain), residues 403-626 of SEQ ID NO: 5 (the colicin Ia cytotoxic domain), residues 451-581 of SEQ ID NO: 6 (the colicin E2 cytotoxic domain), residues 451-576 of SEQ ID NO: 7 (the colicin E7 cytotoxic domain), residues 451-573 of SEQ ID NO: 8 (the colicin E8 cytotoxic domain), SEQ ID NO: 9 (the colicin E4 cytotoxic domain), residues 451-551 of SEQ ID NO: 10 (the colicin E6 cytotoxic domain), SEQ ID NO: 11 (the colicin E5 cytotoxic domain), residues 392-592 of SEQ ID NO: 12 (the colicin A cytotoxic domain), residues 292-511 of SEQ ID NO: 13 (the colicin B cytotoxic domain), residues 187-387 of SEQ ID NO: 14 (the colicin N cytotoxic domain), residues 140-271 of SEQ ID NO: 15 (the colicin M cytotoxic domain), and residues 299-499 of SEQ ID NO: 16 (the colicin S4 cytotoxic domain).

Preferably the colicin has a cytotoxic domain having at least 50%, more preferably 55%, 60%, 65%, 70%, 75%, 80%, 85%, 90%, 95%, 96%, 97%, 98% or 99% amino acid sequence identity to residues 344-522 of SEQ ID NO: 1 (the colicin E1 cytotoxic domain), residues 451-551 of SEQ ID NO. 2 (the colicin E3 cytotoxic domain), residues 451-582 of SEQ ID NO: 3 (the colicin E9 cytotoxic domain), residues 591-697 of SEQ ID NO: 4 (the colicin D cytotoxic domain), or residues 403-626 of SEQ ID NO: 5 (the colicin Ia cytotoxic domain).

The colicin additionally has a receptor-binding domain capable of binding a surface receptor on a target sensitive cell, and a translocation domain capable of directing translocation of the colicin across the outer membrane of a target sensitive cell.

The receptor-binding domain may have an amino acid sequence having at least 50%, more preferably 55%, 60%, 65%, 70%, 75%, 80%, 85%, 90%, 95%, 96%, 97%, 98% or 99% sequence identity to a sequence selected from residues 201-333 of SEQ ID NO: 1 (the colicin E1 receptor-binding domain), residues 316 -450 of SEQ ID NO. 2 (the colicin E3 receptor-binding domain), residues 316-450 of SEQ ID NO: 3 (the colicin E9 receptor-binding domain), residues 299-402 of SEQ ID NO: 5 (the colicin Ia receptor-binding domain), residues 316-450 of SEQ ID NO: 6 (the colicin E2 receptor-binding domain), residues 316-450 of SEQ ID NO: 7 (the colicin E7 receptor-binding domain), residues 316-450 of SEQ ID NO: 8 (the colicin E8 receptor-binding domain), residues 316-450 of SEQ ID NO: 10 (the colicin E6 receptor-binding domain), residues 173-391 of SEQ ID NO: 12 (the colicin A receptor-binding domain), residues 36-139 of SEQ ID NO: 15 (the colicin M receptor-binding domain), and residues 119-299 of SEQ ID NO: 16 (the colicin S4 receptor-binding domain).

Preferably, the receptor-binding domain has an amino acid sequence having at least 50%, more preferably 55%, 60%, 65%, 70%, 75%, 80%, 85%, 90%, 95%, 96%, 97%, 98% or 99% sequence identity to residues 201-333 of SEQ ID NO: 1 (the colicin E1 receptor-binding domain), residues 316 -450 of SEQ ID NO. 2 (the colicin E3 receptor-binding domain), residues 316-450 of SEQ ID NO: 3 (the colicin E9 receptor-binding domain), or residues 299-402 of SEQ ID NO: 5 (the colicin Ia receptor-binding domain).

Alternatively, the colicin may have at least 50%, more preferably 55%, 60%, 65%, 70%, 75%, 80%, 85%, 90%, 95%, 96%, 97%, 98% or 99% amino acid sequence identity to a sequence selected from SEQ ID NO: 1 (colicin E1), SEQ ID NO. 2 (colicin E3), SEQ ID NO: 3 (colicin E9), SEQ ID NO: 4 (colicin D), SEQ ID NO: 5 (colicin Ia), SEQ ID NO: 6 (colicin E2), SEQ ID NO: 7 (colicin E7), SEQ ID NO: 8 (colicin E8), SEQ ID NO: 10 (colicin E6), SEQ ID NO: 12 (colicin A), SEQ ID NO: 13 (colicin B), SEQ ID NO: 14 (colicin N), SEQ ID NO: 15 (colicin M) and SEQ ID NO: 16 (the colicin S4). Sequence identity can be determined by using a standard BLAST alignment using the default parameters.

In another aspect, the invention provides an in vitro method of killing AIEC, of killing bacteria associated with a biofilm, or of killing bacteria in a macrophage, the method comprising contacting the AIEC, the biofilm, or the macrophages with an effective amount of a colicin or an effective quantity of bacteria that produce a colicin.

The invention will now be described in more detail, by way of example and not limitation, by reference to the accompanying drawings. Many equivalent modifications and variations will be apparent to those skilled in the art when given this disclosure. Accordingly, the exemplary embodiments of the invention set forth are considered to be illustrative and not limiting. Various changes to the described embodiments may be made without departing from the scope of the invention.

The present invention includes the combination of the aspects and preferred features described except where such a combination is clearly impermissible or is stated to be expressly avoided. Section headings are used herein are for convenience only and are not to be construed as limiting in any way.

### Description of the Drawings

**Fig 1****. Killing of AI E. coli LF82 growing in the biofilm state by colicins and small molecule antibiotics.** Biofilms of *E. coli* Crohn's isolate LF82 treated with ampicillin, ciprofloxacin, metronidazole, rifaximin and colicins E1, E3, E9 and D for 1h.
**Fig 2****. Survival (%) of LF82 biofilms following treatment with the colicin E1 producing W3110 pColE1-K53 strain and the non-producing W3110 strain.**
**Fig 3****. Killing of intracellular bacteria by colicin E1.**
Fig. 4. **Killing of intracellular bacteria by colicin E9.**

### Detailed Description of the Invention

### Colicins

Colicins can be obtained directly from the bacterial supernatants of colicin producing bacterial strains. Typically, protein is precipitated from the supernatant by ammonium sulphate precipitation and protein purified by ion exchange chromatography and gel filtration. Alternatively, recombinant protein could be produced with an engineered affinity tag, such as a His-tag and the protein purified by affinity chromatography (metal affinity chromatography in the case of His-tagged protein). In some cases in accordance with the invention, the colicin is in a heterodimer with a corresponding immunity protein. Nuclease-type colicin-immunity protein complexes are suitable. In this case, an affinity tag can be engineered onto the immunity protein and the colicin-immunity protein complex isolated by affinity chromatography.

### Bacteria that produce a colicin

Bacterial strains that produce (i.e. express and secrete) a colicin can be isolated from nature. In such cases, the colicin can be regarded as being endogenous to that strain. Alternatively, bacteria that produce (i.e. express and secrete) one or more colicins can be engineered using standard techniques that are well known in the art. Such engineering may be performed to enhance production (e.g. expression and/or secretion) of a colicin already naturally produced by the bacteria, i.e. to enhance production of an endogenous colicin. Alternatively, bacteria may be engineered to produce (i.e. express and secrete) a heterologous colicin, i.e. a colicin which is not naturally produced by those bacteria.

Colicins are typically encoded on plasmids. Thus, bacteria that produce a colicin may be engineered by introducing a plasmid carrying a gene for a colicin, although other expression vectors or constructs may be employed, including chromosomally-integrated expression constructs. Thus the bacterium may comprise an expression vector or expression construct comprising a nucleic acid sequence encoding a colicin, whereby the bacterium is able to express and secrete the colicin. The colicin may be endogenous or heterologous to the bacterium. In some cases the expression vector or construct (e.g. plasmid) additionally encodes (i.e. comprises a nucleic acid sequence encoding) an immunity protein and/or a colicin release protein, whereby the bacterium is able to express said immunity protein and/or release protein, and secrete them if appropriate. An example of a suitable plasmid is pColE1-K53.

Immunity proteins protect the cell from the activity of colicins produced either by the cell itself or by neighbouring cells. Each immunity protein is a specific antagonist of the activity of a corresponding colicin. For nuclease-type colicins, the immunity protein binds strongly to the colicin and prevents it from degrading nucleic acid in the cytoplasm. Typically a colicin-immunity protein complex is released from the producing cell, but the colicin dissociates from the immunity complex before or on entry to a target sensitive cell. Thus, in some cases in accordance with the invention, the bacterium produces both a colicin, for example a nuclease-type colicin, and a corresponding immunity protein. The bacterium may release a colicin-immunity protein complex.

Bacteria that produce a pore forming colicin may also produce a corresponding immunity protein. For pore-forming colicins, the immunity protein is typically an inner membrane protein that prevents exogenous colicin from depolarising the membrane. Cytoplasmic inophoric colicin is not active on the producing cell. Thus, in some cases in accordance with the invention, the bacterium produces both a pore-forming colicin and a corresponding immunity protein. In other cases, the bacterium that produces the pore-forming colicin does not express the receptor that is recognised by the colicin, so the producing cell is not sensitive to exogenous colicin made by neighbouring cells of the same strain. Example immunity proteins are disclosed in Cascales et al. (2007) and Papadakos et al (2011).

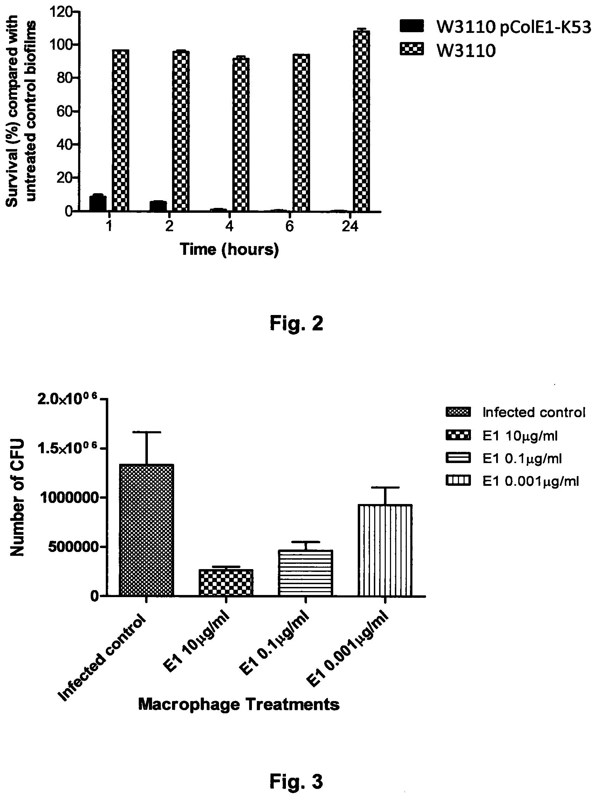

The colicin may be released from the bacterium by any suitable means. For example, a suitable targeting sequence may be engineered onto the colicin to direct its secretion from the cell. Alternatively, the colicin producing bacterium may co-express a colicin release protein. Colicin release proteins, also known as colicin lysis proteins, are small lipoproteins having a common consensus sequence that are sometimes co-expressed with a colicin. The release of colicins from a cell that co-expresses a colicin release protein occurs through a unique mechanism. Expression of the colicin release protein is lethal to the producing cell. The amino acid sequences of example lysis release proteins are disclosed in Fig. 2 of Cascales et al. (2007).

### Probiotics

A living microorganism that confers a health benefit when administered or consumed in adequate amounts is referred to as a probiotic, or a probiotic organism. A probiotic organism can be used as an ingredient in a food product or in a diet supplement. In some cases, the bacterium in accordance with the invention is a probiotic bacterium.

### Subjects for treatment

Preferred subjects for treatment by the methods of the invention are mammals. Preferred subjects are primates (including humans), rodents (including mice and rats), and other common laboratory, domestic and agricultural animals, including but not limited to rabbits, dogs, cats, horses, cows, sheep, goats, etc.

### Pharmaceutical compositions and methods of treatment

The colicins and bacteria described herein can be formulated in pharmaceutical compositions. These compositions may comprise, in addition to one of the above substances, a pharmaceutically acceptable excipient, carrier, buffer, stabiliser or other materials well known to those skilled in the art. Such materials should be non-toxic and should not interfere with the efficacy of the active ingredient. The precise nature of the carrier or other material may depend on the route of administration, e.g. oral, intravenous, cutaneous or subcutaneous, nasal, intramuscular and intraperitoneal routes. Examples of suitable compositions and methods of administration are provided in Esseku and Adeyeye (2011) and Van den Mooter G. (2006).

Preferably the route of administration is oral. Pharmaceutical compositions for oral administration may be in tablet, capsule, powder or liquid form. A tablet may include a solid carrier such as gelatin or an adjuvant. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required. Whatever the nature of the active agent that is to be given to an individual (e.g. a cell, polypeptide, nucleic acid molecule, other pharmaceutically useful agent according to the present invention), administration is preferably in a "prophylactically effective amount" or a "therapeutically effective amount" (as the case may be, although prophylaxis may be considered therapy), this being sufficient to show benefit to the individual. The actual amount administered, and rate and time-course of administration, will depend on the nature and severity of what is being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 20th Edition, 2000, pub. Lippincott, Williams & Wilkins.

A composition may be administered alone or in combination with other treatments, including other colicins or colicin producing bacteria, either simultaneously or sequentially dependent upon the condition to be treated.

### Sequences

SEQ ID NO: 1
   Colicin E1 (UniProt: P02978, Last modified July 21, 1986. Version 1. Checksum: A77C351BBC1AB7C1)
   Domain structure: T-domain 1-200, R-domain 201-333, C-domain 334-522 
METATAVAYYKDGVPYDDKGQVIITLLNGTPDGSGSGGGGGKGGSKSESSAAIHATAKWSTAQLKKT
QAEQAARAKAAAEAQAKAKANRDALTQRLKDIVNEALRHNASRTPSATELAHANNAAMQAEDERL
RLAKAEEKARKEAEAAEKAFQEAEQRRKEIEREKAETERQLKLAEAEEKRLAALSEEAKAVEIAQ
KKLSAAQSEVVKMDGEIKTLNSRLSSSIHARDAEMKTLAGKRNELAQASAKYKELDELVKKLSPR
ANDPLQNRPFFEATRRRVGAGKIREEKQKQVTASETRINRINADITQIQKAISQVSNNRNAGIAR
VHEAEENLKKAQNNLLNSQIKDAVDATVSFYQTLTEKYGEKYSKMAQELADKSKGKKIGNVNEAL
AAFEKYKDVLNKKFSKADRDAIFNALASVKYDDWAKHLDQFAKYLKITGHVSFGYDVVSDILKIK
DTGDWKPLFLTLEKKAADAGVSYVVALLFSLLAGTTLGIWGIAIVTGILCSYIDKNKLNTINEVL
GI
SEQ ID NO: 2
   Colicin E3 (Uniprot: P00646, Last modified April 1, 1988. Version 1. Checksum: E444CE918D89ECD6)
   Domain structure: T-domain 1-315, R-domain 316 -450, C-domain 451-551 
MSGGDGRGHNTGAHSTSGNINGGPTGLGVGGGASDGSGWSSENNPWGGGSGSGIHWGGGSGHGNG
GGNGNSGGGSGTGGNLSAVAAPVAFGFPALSTPGAGGLAVSISAGALSAAIADIMAALKGPFKFG
LWGVALYGVLPSQIAKDDPNMMSKIVTSLPADDITESPVSSLPLDKATVNVNVRVVDDVKDERQN
ISVVSGVPMSVPVVDAKPTERPGVFTASIPGAPVLNISVNNSTPAVQTLSPGVTNNTDKDVRPAG
FTQGGNTRDAVIRFPKDSGHNAVYVSVSDVLSPDQVKQRQDEENRRQQEWDATHPVEAAERNYER
ARAELNQANEDVARNQERQAKAVQVYNSRKSELDAANKTLADAIAEIKQFNRFAHDPMAGGHRMW
QMAGLKAQRAQTDVNNKQAAFDAAAKEKSDADAALSSAMESRKKKEDKKRSAENNLNDEKNKPRK
GFKDYGHDYHPAPKTENIKGLGDLKPGIPKTPKQNGGGKRKRWTGDKGRKIYEWDSQHGELEGYR
ASDGQHLGSFDPKTGNQLKGPDPKRNIKKYL
SEQ ID NO: 3
   Colicin E9 (Uniprot: P09883, Last modified October 1, 1996. Version 4. Checksum: 47A71B57B45AFDD9)
   Domain structure: T-domain 1-315, R-domain 316 -450, C-domain 451-582 
MSGGDGRGHNTGAHSTSGNINGGPTGIGVSGGASDGSGWSSENNPWGGGSGSGIHWGGGSGRGNG

GGNGNSGGGSGTGGNLSAVAAPVAFGFPALSTPGAGGLAVSISASELSAAIAGIIAKLKKVNLKF

TPFGVVLSSLIPSEIAKDDPNMMSKIVTSLPADDITESPVSSLPLDKATVNVNVRVVDDVKDERQ

NISVVSGVPMSVPVVDAKPTERPGVFTASIPGAPVLNISVNDSTPAVQTLSPGVTNNTDKDVRPA

GFTQGGNTRDAVIRFPKDSGHNAVYVSVSDVLSPDQVKQRQDEENRRQQEWDATHPVEAAERNYE
 
RARAELNQANEDVARNQERQAKAVQVYNSRKSELDAANKTLADAIAEIKQFNRFAHDPMAGGHRM
WQMAGLKAQRAQTDVNNKQAAFDAAAKEKSDADAALSAAQERRKQKENKEKDAKDKLDKESKRNK
PGKATGKGKPVGDKWLDDAGKDSGAPIPDRIADKLRDKEFKSFDDFRKAVWEEVSKDPELSKNLN
PSNKSSVSKGYSPFTPKNQQVGGRKVYELHHDKPISQGGEVYDMDNIRVTTPKRHIDIHRGK
SEQ ID NO: 4
   Colicin D (Uniprot: P17998, Last modified November 1, 1990. Version 1. Checksum: F468B4A0172ABBB1)Domain structure: C-domain 591-697 
MSDYEGSGPTEGIDYGHSMVVWPSTGLISGGDVKPGGSSGIAPSMPPGWGDYSPQGIALVQSVLF
PGIIRRIILDKELEEGDWSGWSVSVHSPWGNEKVSAARTVLENGLRGGLPEPSRPAAVSFARLEP
ASGNEQKIIRLMVTQQLEQVTDIPASQLPAAGNNVPVKYRLMDLMQNGTQYMAIIGGIPMTVPVV
DAVPVPDRSRPGTNIKDVYSAPVSPNLPDLVLSVGQMNTPVLSNPEIQEEGVIAETGNYVEAGYT
MSSNNHDVIVRFPEGSDVSPLYISTVEILDSNGLSQRQEAENKAKDDFRVKKEEAVARAEAEKAK
AELFSKAGVNQPPVYTQEMMERANSVMNEQGALVLNNTASSVQLAMTGTGVWTAAGDIAGNISKF
FSNALEKVTIPEVSPLLMRISLGALWFHSEEAGAGSDIVPGRNLEAMFSLSAQMLAGQGVVIEPG
ATSVNLPVRGQLINSNGQLALDLLKTGNESIPAAVPVLNAVRDTATGLDKITLPAVVGAPSRTIL
VNPVPQPSVPTDTGNHQPVPVTPVHTGTEVKSVEMPVTTITPVSDVGGLRDFIYWRPDAAGTGVE
AVYVMLNDPLDSGRFSRKQLDKKYKHAGDFGISDTKKNRETLTKFRDAIEEHLSDKDTVEKGTYR
REKGSKVYFNPNTMNVVIIKSNGEFLSGWKINPDADNGRIYLETGEL
SEQ ID NO: 5
   Colicin Ia (UniProt: P06716, Last modified October 24, 2003. Version 2. Checksum: 3DCODF322F405D39)
   Domain structure: T domain 1-298, R domain 299-402, C domain 403-626 
MSDPVRITNPGAESLGYDSDGHEIMAVDIYVNPPRVDVFHGTPPAWSSFGNKTIWGGNEWVDDSP
TRSDIEKRDKEITAYKNTLSAQQKENENKRTEAGKRLSAAIAAREKDENTLKTLRAGNADAADIT
RQEFRLLQAELREYGFRTEIAGYDALRLHTESRMLFADADSLRISPREARSLIEQAEKRQKDAQN
ADKKAADMLAEYERRKGILDTRLSELEKNGGAALAVLDAQQARLLGQQTRNDRAISEARNKLSSV
TESLNTARNALTRAEQQLTQQKNTPDGKTIVSP[?]KFPGRSSTNHSIVVSGDPRFAGTIKITTSAV
IDNRANLNYLLSHSGLDYKRNILNDRNPVVTEDVEGDKKIYNAEVAEWDKLRQRLLDARNKITSA
ESAVNSARNNLSARTNEQKHANDALNALLKEKENIRNQLSGINQKIAEEKRKQDELKATKDAINF
TTEFLKSVSEKYGAKAEQLAREMAGQAKGKKIRNVEEALKTYEKYRADINKKINAKDRAAIAAAL
 
ESVKLSDISSNLNRFSRGLGYAGKFTSLADWITEFGKAVRTENWRPLFVKTETIIAGNAATALVA
LVFSILTGSALGIIGYGLLMAVTGALIDESLVEKANKFWGI
SEQ ID NO: 6
   Colicin E2 (UniProt: B5TQU9, Last modified November 4, 2008. Version 1.
   Checksum: B046C6FC7BF0FE2C)
   Domain structure: T-domain 1-315, R-domain 316-450, C-domain 451-581 
MSGGDGRGHNTGAHSTSGNINGGPTGLGVGGGASDGSGWSSENNPWGGGSGSGIHWGGGSGHGNG
GGNSNSGGGSGTGGNLSAVAAPVAFGFPALSTPGAGGLAVSISAGALSAAIADIMAALKGPFKFG
LWGVALYGVLPSQIAKDDPNMMSKIVTSLPADDITESPVSSLPLDKATVNVNVRVVDDVKDERQN
ISVVSGVPMSVPVVDAKPTERPGVFTASIPGAPVLNISVNNSTPAVQTLSPGVTNNTDKDVRPAG
FTQGGNTRDAVIRFPKDSGHNAVYVSVSDVLSPDQVKQRQDEENRRQQEWDATHPVEAAERNYER
ARAELNQANEDVARNQERQAKAVQVYNSRKSELDAANKTLADAIAEIKQFDRFAHDPMAGGHRMW
QMAGLKAQRAQTDVNNKQAAFDAAAKEKSDADAALSAAQERRKQKENKEKDAKDKLDKESKRNKP
GKATGKGKPVGDKWLDDAGKDSGAPIPDRIADKLRDKEFKNFDDFRKKFWEEVSKDPDLSKQFKG
SNKTNIQKGKAPFARKKDQVGGRERFELHHDKPISQDGGVYDMNNIRVTTPKRHIDIHRGK
SEQ ID NO: 7
   Colicin E7 (Uniprot: Q47112, Last modified May 30, 2000. Version 2. Checksum: E5B05E73B2E17249)
   Domain structure: T-domain 1-315, R-domain 316-450, C-domain 451-576 
MSGGDGRGHNSGAHNTGGNINGGPTGLGGNGGASDGSGWSSENNPWGGGSGSGVHWGGGSGHGNG
GGNSNSGGGSNSSVAAPMAFGFPALAAPGAGTLGISVSGEALSAAIADIFAALKGPFKFSAWGIA
LYGILPSEIAKDDPNMMSKIVTSLPAETVTNVQVSTLPLDQATVSVTKRVTDVVKDTRQHIAVVA
GVPMSVPVVNAKPTRTPGVFHASFPGVPSLTVSTVKGLPVSTTLPRGITEDKGRTAVPAGFTFGG
GSHEAVIRFPKESGQKPVYVSVTDVLTPAQVKQRQDEEKRLQQEWNDAHPVEVAERNYEQARAEL
NQANKDVARNQERQAKAVQVYNSRKSELDAANKTLADAKAEIKQFERFAREPMAAGHRMWQMAGL
KAQRAQTDVNNKKAAFDAAAKEKSDADVALSSALERRKQKENKEKDAKAKLDKESKRNKPGKATG
KGKPVNNKWLNNAGKDLGSPVPDRIANKLRDKEFKSFDDFRKKFWEEVSKDPELSKQFSRNNNDR
MKVGKAPKTRTQDVSGKRTSFELHHEKPISQNGGVYDMDNISVVTPKRHIDIHRGK
SEQ ID NO: 8
   Colicin E8 (UniProt: C6GJY4, Last modified September 1, 2009. Version 1. Checksum: 42ECBBDB92EODB52)
   Domain structure: T domain 1-315, R domain 316-450, C domain 451-573 
MSGGDGRGHNTGAHSTSGNINGGPTGIGVSGGASDGSGWSSENNPWGGGSGSGIHWGGGSGRGNG
GGNGNSGGGSGTGGNLSAVAAPVAFGFPALSTPGAGGLAVSISASELSAAIAGIIAKLKKVNLKF
TPFGVVLSSLIPSEIAKDDPNMMSKIVTSLPADDITESPVSSLPLDKATVNVNVRVVDDVKDERQ
NISVVSGVPMSVPVVDAKPTERPGVFTASIPGAPVLNISVNNSTPAVQTLSPGVTNNTDKDVRPA
GFTQGGNTRDAVIRFPKDSGHNAVYVSVSDVLSPDQVKQRQDEENRRQQEWDATHPVEAAERNYE
RARAELNQANEDVARNQERQAKAVQVYNSRKSELDAANKTLADAIAEIKQFNRFAHDPMAGGHRM
WQMAGLKAQRAQTDVNNKQAADADAALSAAQERRKQKENKEKDAKDKLDKESKRNKPGKATGKGK
PVGDKWLDDAGKDSGAPIPDRIADKLRDKEFKNFDDFRRKFWEEVSKDPELSKQFNPGNKKRLSQ
GLAPRARNKDTVGGRRSFELHHDKPISQDGGVYDMDNLRITTPKRHIDIHRGQ
SEQ ID NO: 9
   Colicin E4 Cytotoxic domain (Uniprot: Q47109, Last modified November 1, 1996. Version 1. Checksum: 6F997EED8BF568D1) 
ERFAREPMAAGHRMWQMAGLKAQRAQTDVNNKKAAFDAAAKEKSDADAALSSAMESRKKKEDKKR
SAENKLNEEKNKPRKGVKDYGHDYHPAPKTEEIKGLGELKKAPKKTPKQGGGGRRDRWIGDKGRK
IYEWDSQHGELEGYRASDGEHIGAFDPKTGKQIKGPDPKGRNIKKYL
SEQ ID NO: 10
   Colicin E6 (UniProt: P17999, Last modified November 1, 1990. Version 1. Checksum: D223D7F0770392EO)
   Domain structure: T-domain 1-315, R-domain 316-450, C-domain 451-551 
MSGGDGRGHNTGAHSTSGNINGGPTGLGVGGGASDGSGWSSENNPWGGGSGSGIHWGGGSGHGNG
GGNGNSGGGSGTGGNLSAVAAPVAFGFPALSTPGAGGLAVSISAGALSAAIADIMAALKGPFKFG
LWGVALYGVLPSQIAKDDPNMMSKIVTSLPADDITESPVSSLPLDKATVNVNVRVVDDVKDERQN
ISVVSGVPMSVPVVDAKPTERPGVFTASIPGAPVLNISVNNSTPAVQTLSPGVTNNTDKDVRPAG
FTQGGNTRDAVIRFPKDSGHNAVYVSVSDVLSPDQVKQRQDEENRRQQEWDATHPVEAAERNYER
ARAELNQANEDVARNQERQAKAVQVYNSRKSELDAANKTLADAIAEIKQFNRFAHDPMAGGHRMW
QMAGLKAQRAQTDVNNKQAAFDAAAKEKSDADAALSSAMESRKKKEDKKRSAENKLNEEKNKPRK
GVKDYGHDYHPDPKTEDIKGLGELKEGKPKTPKQGGGGKRARWYGDKGRKIYEWDSQHGELEGYR
ASDGQHLGSFEPKTGNQLKGPDPKRNIKKYL
SEQ ID NO: 11
   Colicin E5 cytotoxic domain (UniProt: P18000, Last modified November 1, 1990. Version 1. Checksum: 9DE1713F474B2BA4) 
RFAHDPMAGGHRMWQMAGLKAQRAQTDVNNKQAAFDAAAKEKADADAALSTAMESRKKKEDNKRD
AEGKLNDELAKNKGKIPGLKIDQKIRGQMPERGWTEDDIKNTVSNGATGTSFDKRSPKKTPPDYL
GRNDPATVYGSPGKYVVVNDRTGEVTQISDKTDPGWVDDSRIQWGNKNDQ
SEQ ID NO: 12
   Colicin A (UniProt: P04480, Last modified August 13, 1987. Version 1. Checksum: B80FA1F52A8CFC5D)
   Domain structure: T domain 1-172, R domain 173-391, C domain 392-592 
MPGFNYGGKGDGTGWSSERGSGPEPGGGSHGNSGGHDRGDSSNVGNESVTVMKPGDSYNTPWGKV
IINAAGQPTMNGTVMTADNSSMVPYGRGFTRVLNSLVNNPVSPAGQNGGKSPVQTAVENYLMVQS
GNLPPGYWLSNGKVMTEVREERTSGGGGKNGNERTWTVKVPREVPQLTASYNEGMRIRQEAADRA
RAEANARALAEEEARAIASGKSKAEFDAGKRVEAAQAAINTAQLNVNNLSGAVSAANQVITQKQA
EMTPLKNELAAANQRVQETLKFINDPIRSRIHFNMRSGLIRAQHNVDTKQNEINAAVANRDALNS
QLSQANNILQNARNEKSAADAALSAATAQRLQAEAALRAAAEAAEKARQRQAEEAERQRQAMEVA
EKAKDERELLEKTSELIAGMGDKIGEHLGDKYKAIAKDIADNIKNFQGKTIRSFDDAMASLNKIT
ANPAMKINKADRDALVNAWKHVDAQDMANKLGNLSKAFKVADVVMKVEKVREKSIEGYETGNWGP
LMLEVESWVLSGIASSVALGIFSATLGAYALSLGVPAIAVGIAGILLAAVVGALIDDKFADALNN
EIIRPAH
SEQ ID NO: 13
   Colicin B (UniProt: P05819, Last modified January 23, 2007. Version 3. Checksum: 8ABB972CF1925964)
   Domain structure: T and R domains 1-291, C domain 292-511 
MSDNEGSVPTEGIDYGDTMVVWPSTGRIPGGDVKPGGSSGLAPSMPPGWGDYSPQGIALVQSVLF
PGIIRRIILDKELEEGDWSGWSVSVHSPWGNEKVSAARTVLENGLRGGLPEPSRPAAVSFARLEP
ASGNEQKIIRLMVTQQLEQVTDIPASQLPAAGNNVPVKYRLTDLMQNGTQYMAIIGGIPMTVPVV
DAVPVPDRSRPGTNIKDVYSAPVSPNLPDLVLSVGQMNTPVRSNPEIQEDGVISETGNYVEAGYT
MSSNNHDVIVRFPEGSGVSPLYISAVEILDSNSLSQRQEAENNAKDDFRVKKEQENDEKTVLTKT
SEVIISVGDKVGEYLGDKYKALSREIAENINNFQGKTIRSYDDAMSSINKLMANPSLKINATDKE
 
AIVNAWKAFNAEDMGNKFAALGKTFKAADYAIKANNIREKSIEGYQTGNWGPLMLEVESWVISGM
ASAVALSLFSLTLGSALIAFGLSATVVGFVGVVIAGAIGAFIDDKFVDELNHKIIK
SEQ ID NO: 14
   Colicin N (UniProt: P08083, Last modified August 1, 1988. Version 1. Checksum: 1C4342E222F8CECD)
   Domain structure: T and R domains 1-186, C domain 187-387 
MGSNGADNAHNNAFGGGKNPGIGNTSGAGSNGSASSNRGNSNGWSWSNKPHKNDGFHSDGSYHIT
FHGDNNSKPKPGGNSGNRGNNGDGASAKVGEITITPDNSKPGRYISSNPEYSLLAKLIDAESIKG
TEVYTFHTRKGQYVKVTVPDSNIDKMRVDYVNWKGPKYNNKLVKRFVSQFLLFRKEEKEKNEKEA
LLKASELVSGMGDKLGEYLGVKYKNVAKEVANDIKNFHGRNIRSYNEAMASLNKVLANPKMKVNK
SDKDAIVNAWKQVNAKDMANKIGNLGKAFKVADLAIKVEKIREKSIEGYNTGNWGPLLLEVESWI
IGGVVAGVAISLFGAVLSFLPISGLAVTALGVIGIMTISYLSSFIDANRVSNINNIISSVIR
SEQ ID NO: 15
   Colicin M (Uniprot P05820, Last modified November 1, 1988. Version 1. Checksum: B41B7BE107EC1DBA)
   Domain structure: T domain 1-35, R domain 36-139, C domain 140-271 
METLTVHAPSPSTNLPSYGNGAFSLSAPHVPGAGPLLVQVVYSFFQSPNMCLQALTQLEDYIKKH
GASNPLTLQIISTNIGYFCNADRNLVLHPGISVYDAYHFAKPAPSQYDYRSMNMKQMSGNVTTPI
VALAHYLWGNGAERSVNIANIGLKISPMKINQIKDIIKSGVVGTFPVSTKFTHATGDYNVITGAY
LGNITLKTEGTLTISANGSWTYNGVVRSYDDKYDFNASTHRGIIGESLTRLGAMFSGKEYQILLP
GEIHIKESGKR
SEQ ID NO: 16
   Colicin S4 (UniProt: Q9XB47, Last modified November 1, 1999. Version 1. Checksum: 3E36C7271BF1D293)
   Domain structure: T domain 1-118, R domain 119-299, C domain 299-499

MAKELSVYGPTAGESMGGTGANLNQQGGNNNSNSGVHWGGGSGSGNGGREHGSQTGWGWSKTNNP
DVPPYVDDNGQVRITITNGLVKTPVYGVPGAGGNSDVQGGYIPENPNDEVARKWDKNNLPREIDV
SIDGFKYRVTLNDNGRAIGILRTGVRPYVGSEKAKAGIMEKINHKTPEEIYEALGFNKDESQRQE
KAKQQAEDAWDRLPPNVRKFDVDVEQFHYLVVLDDYGNVLSVTRTGVRPYVGSEKAKAGIMDKVD
HKTPEEIYEALGFNNEEPQRQNQAKKAAYDVFYSFSMNRDRIQSDVLNKAAEVISDIGNKVGDYL

GDAYKSLAREIADDVKNFQGKTIRSYDDAMASLNKVLSNPGFKFNRADSDALANVWRSIDAQDMA
NKLGNISKAFKFADVVMKVEKVREKSIEGYET[?]NWGPLMLEVESWVLSGIASAVALGVFSATLGA
YALSLGAPAIAVGIVGILLAAVVGALLDDKFADALNKEIIKPAH

### EXAMPLES

### Example 1

### Activity of colicins against AI E. coli

Four adherent invasive *E. coli* isolates (AIEC) recovered from patients with Crohns' disease (isolates 95, 419, 615 and the AIEC type strain LF82) were shown to be sensitive to colicins E1, E3, E9 and D in spot tests, where purified colicin is spotted onto a growing lawn of cells.

Briefly, 25 µl of a log phase bacterial culture (OD₆₀₀=0.6) was added to 5 ml of molten 0.8% agarose and poured on top of an LB agar plate. A 2 µl drop of each colicin (0.2 mg/ml) was spotted onto the overlay plate. The plates were incubated for 18 h and examined for zones of inhibition. All *E. coli* isolates tested were sensitive to the cytotoxic activity of the four colicin proteins, as indicated by the presence of clear zones representing cell killing (not shown).

### Example 2

### The activity of a colicin E1-producing strain against AIEC biofilms

The clinically relevant state for AI *E. coli* in the infected gut mucosa is the biofilm state in which bacteria show enhanced tolerance to antibiotics. We therefore tested the ability of colicins to kill AI *E. coli* in the biofilm state using the MBEC Physiology and Genetics Assay (Innovotech). In this assay, biofilm growth occurs on 96 identical pegs protruding from the lid of a 96-well microtitre plate. Biofilm formation was demonstrated using electron microscopy (not shown).

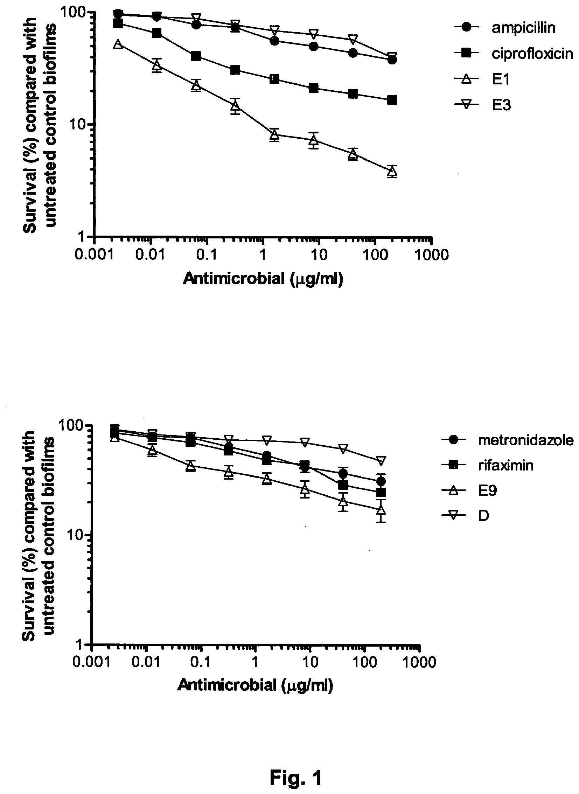

To determine the cytotoxicity of colicins against LF82 biofilms we compared the % cell survival of AIEC in biofilms treated with colicins with those exposed to antibiotics which are frequently prescribed in the treatment of Crohn's disease (Figure 1). Biofilms were formed on the MBEC™ 96-peg plate platform for 24h, then challenged for 1 h with 150 µl dilutions of the antibiotics (ampicillin, ciprofloxacin, metronidazole and rifaximin) and colicins (E1, E3, E9 and D) in sterile PBS in the concentration range 0.002 µg/ml - 200 µg/ml. All dilutions of antimicrobials were done in triplicate and control antimicrobial-free biofilms were treated with 150 µl of sterile PBS. Following treatment, the viability of biofilm-associated cells was tested using the metabolic dye XTT. The XTT salt changes colour when it is metabolised by viable cells. This colour change can be compared to untreated antimicrobial-free biofilms, giving an indication of the percentage survival in biofilms exposed to the antimicrobials.

We further tested the ability of colicins E1 (a pore-forming colicin) E3 (a rRNase), E9 (a DNase), D (a tRNase) and IA (a pore-forming colicin) to kill 6 additional strains of AIEC in the biofilm state. All showed good killing activity against the multiple strains. Colicins E1 (a membrane depolarising colicin) and E9 (a DNase colicin) were the most effective in killing AIEC in the biofilm state. For all isolates, colicin E1 and E9 displayed superior antimicrobial activity against the biofilm-associated cells than the antibiotics ampicillin, metronidazole and rifaximin.

### Example 3

### The activity of a colicin E1-producing strain against AIEC biofilms

We envisage that colicins could be delivered in the form of a colicin producing bacterial strain. To determine if the addition of a colicin producing E. coli strain to LF82 resulted in killing of biofilm associated bacteria we added E.coli K-12 W3110 carrying the colicin E1 plasmid pColE1-K53 to 24 hour LF82 biofilms.

Biofilms of the LF82 strain were formed for 24h on the MBEC™ 96-peg plate platform. A 150µl culture volume of W3110 pColE1-K53 was also grown in the wells of a 96-well flat-bottom plate in LB broth supplemented with a sub-inhibitory concentration of the antibiotic ciprofloxacin (0.001µg/ml). Ciprofloxicin induces colicin production through activation of the SOS response to DNA damage. The antibiotic induces the expression of the colicin E1 protein in this *E. coli* K12 strain. The 24h LF82 biofilms were exposed to the W3110 pColE1-K53 strain for 1, 2, 4, 6, and 24h. The pegs with the treated LF82 biofilms were then removed from the plate, placed in 1ml of sterile PBS, sonicated and vortexed to remove the bacteria from the surface. The sonicate was plated out on LB agar containing 50µg/ml ampicillin to select for colonies of LF82, which are resistant to ampicillin. The plates were incubated for 18h and colonies were counted to determine the survival of LF82 biofilm-associated cells following treatment with the K-12 colicin E1-producing strain. As a control, biofilms of LF82 were also exposed to the non-colicin-producing K-12 strain W3110 to ensure that the presence of the K-12 strain did not affect LF82 cell viability.

Addition of the colicin producing strain W3110 pColE1-K53 greatly reduced survival of LF82, whereas addition of W3110 lacking the colicin E1 plasmid had no effect on survival of the AI E. coli strain (Figure 2).

### Example 4

### The activity of colicins E1 and E9 in a macrophage model of AIEC infection

AI *E. coli* are able to infect and replicate within macrophages and epithelial cells. To determine if colicin E1 is able to kill intracellular bacteria raw murine macrophages (J774) were infected with AI E. coli LF82 and treated with colicin E1 (Figure 3) .

Raw murine macrophages (J774) were grown in 24-well tissue culture plates in RPMI tissue culture media (containing 3% fetal calf serum, 1% L-glutamate, 1% pen/strep) at 37°C in 5% CO₂ until the cells reached 70-80% confluency. The macrophages were then infected with *E. coli* LF82 at a multiplicity of infection of 50 (MOI 50). After 2h of infection, the macrophages were exposed to the antibiotic gentamicin (100µg/ml) to kill any bacteria outside the macrophage which hadn't successfully invaded the cell. The LF82-infected macrophages were then treated with colicin E1 (10, 0.1, 0.001µg/ml) for 3h at 37°C. The macrophages were then scraped from the surface of the plate, lysed with Triton X and the intra-cellular bacteria were plated on LB agar containing 50µg/ml ampicillin for selection of LF82. The plates were incubated overnight at 37°C and colony counts were performed. Treatment with colicin E1 (10µg/ml) resulted in approximately an 80% reduction in the number of bacteria recovered from the macrophages showing that this colicin can kill intracellular bacteria.

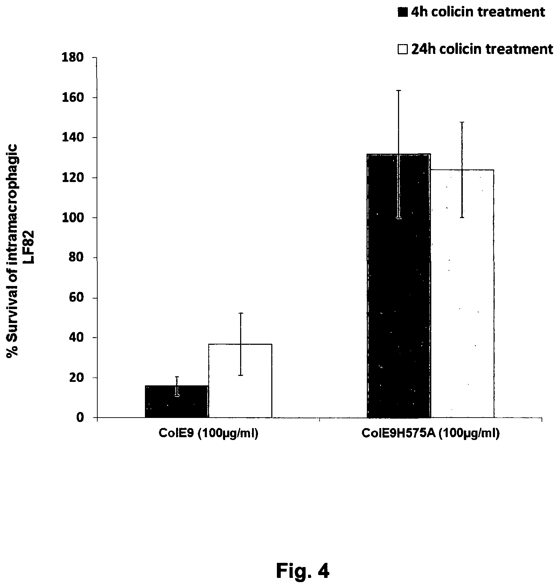

We also tested the ability of colicin E9 and a catalytically inactive colicin E9 variant (colicin E9 H575A) for the ability to kill intracellular *E. coli* LF82 in infected macrophages. Experiments were performed as described for colicin E1 except that infected macrophages were treated with colicin for 4 and 24 hours. Like colicin E1, wild-type colicin E9 was able to kill intracellular LF82 but in infected macrophages treated with the catalytically inactive mutant colicin E9 H575A no killing of intracellular bacteria was observed. See Figure 4. These data indicate that killing is a direct consequence of the cytotoxic activity of the colicin and not through activation of host (macrophage) cell killing pathways.

Fluorescence microscopy has been used to visualise infected macrophages treated with colicin E9 tagged with red fluorescent protein. These studies clearly show internalisation of colicin E9 into the macrophages, further reinforcing our findings that colicins are capable of entering macrophage cells and acting on intracellular bacteria. (Data not shown.)

### EQUIVALENTS

The foregoing written specification is considered to be sufficient to enable one skilled in the art to practice the invention. The present invention is not to be limited in scope by examples provided, since the examples are intended as a single illustration of one aspect of the invention and other functionally equivalent embodiments are within the scope of the invention. Various modifications of the invention in addition to those shown and described herein will become apparent to those skilled in the art from the foregoing description and fall within the scope of the appended claims. The advantages and objects of the invention are not necessarily encompassed by each embodiment of the invention.

### References

1. Loftus SR, Walker D, Mate MJ, Bonsor DA, James R, Moore GR, Kleanthous C. Competitive recruitment of the periplasmic translocation portal TolB by a natively disordered domain of colicin E9. Proc Natl Acad Sci USA, 103(33):12353-82006 (2006)
2. Walker D, Mosbahi K, Vankemmelbeke M, James R, Kleanthous C. The role of electrostatics in colicin nuclease domain translocation into bacterial cells. J Biol Chem., 282(43):31389-97 (2007)
3. Housden NG, Loftus SR, Moore GR, James R, Kleanthous C. Cell entry mechanism of enzymatic bacterial colicins: porin recruitment and the thermodynamics of receptor binding. Proc Natl Acad Sci USA., 27;102(39):13849-54 (2005).
4. Boudeau J, Glasser AL, Masseret E, Joly B, Darfeuille-Michaud A. Invasive ability of an Escherichia coli strain isolated from the ileal mucosa of a patient with Crohn's disease. Infect Immun., 67(9):4499-509 (1999)
5. Carvalho FA, Barnich N, Sauvanet P, Darcha C, Gelot A, Darfeuille-Michaud A. Crohn's disease-associated Escherichia coli LF82 aggravates colitis in injured mouse colon via signaling by flagellin. Inflamm Bowel Dis., 14(8):1051-60 (2008).
6. Carvalho FA, Barnich N, Sivignon A, Darcha C, Chan CH, Stanners CP, Darfeuille-Michaud A. Crohn's disease adherent-invasive Escherichia coli colonize and induce strong gut inflammation in transgenic mice expressing human CEACAM. J Exp Med., 206(10):2179-89 (2009)
7. Miquel S, Peyretaillade E, Claret L, de Vallée A, Dossat C, Vacherie B, Zineb el H, Segurens B, Barbe V, Sauvanet P, Neut C, Colombel JF, Medigue C, Mojica FJ, Peyret P, Bonnet R, Darfeuille-Michaud A. Complete genome sequence of Crohn's disease-associated adherent-invasive E. coli strain LF82. PLoS One., 5(9). pii: e12714 (2010).
8. Martinez-Medina M, Naves P, Blanco J, Aldeguer X, Blanco JE, Blanco M, Ponte C, Soriano F, Darfeuille-Michaud A, Garcia-Gil LJ. Biofilm formation as a novel phenotypic feature of adherent-invasive Escherichia coli (AIEC). BMC Microbiol., 9:202 (2009).
9. Glasser AL, Boudeau J, Barnich N, Perruchot MH, Colombel JF, Darfeuille-Michaud A. Adherent invasive Escherichia coli strains from patients with Crohn's disease survive and replicate within macrophages without inducing host cell death. Infect Immun., 69 (9) :5529-37 (2001).
10. Prantera C, Scribano ML. Antibiotics and probiotics in inflammatory bowel disease: why, when, and how. Curr Opin Gastroenterol.; 25(4) : 329-33 (2009).
11. Rolfe VE, Fortun PJ, Hawkey CJ, Bath-Hextall F. Probiotics for maintenance of remission in Crohn's disease. Cochrane Database Syst Rev., (4):CD004826 (2006).
12. Martinez-Medina M, Mora A, Blanco M, López C, Alonso MP, Bonacorsi S, Nicolas-Chanoine MH, Darfeuille-Michaud A, Garcia-Gil J, Blanco J. Similarity and divergence among adherent-invasive Escherichia coli and extraintestinal pathogenic E. coli strains. J Clin Microbiol., 47(12):3968-79 (2009)
13. Barrett JC, Hansoul S, Nicolae DL, Cho JH, Duerr RH, Rioux JD, Brant SR, Silverberg MS, Taylor KD, Barmada MM, Bitton A, Dassopoulos T, Datta LW, Green T, Griffiths AM, Kistner EO, Murtha MT, Regueiro MD, Rotter JI, Schumm LP, Steinhart AH, Targan SR, Xavier RJ; NIDDK IBD Genetics Consortium, Libioulle C, Sandor C, Lathrop M, Belaiche J, Dewit O, Gut I, Heath S, Laukens D, Mni M, Rutgeerts P, Van Gossum A, Zelenika D, Franchimont D, Hugot JP, de Vos M, Vermeire S, Louis E; Belgian-French IBD Consortium; Wellcome Trust Case Control Consortium, Cardon LR, Anderson CA, Drummond H, Nimmo E, Ahmad T, Prescott NJ, Onnie CM, Fisher SA, Marchini J, Ghori J, Bumpstead S, Gwilliam R, Tremelling M, Deloukas P, Mansfield J, Jewell D, Satsangi J, Mathew CG, Parkes M, Georges M, Daly MJ. Genome-wide association defines more than 30 distinct susceptibility loci for Crohn's disease. Nat Genet. 40(8):955-62 (2008)
14. Cascales E, Buchanan SK, Duché D, Kleanthous C, Lloubès R, Postle K, Riley M, Slatin S, Cavard D. Colicin biology. Microbiol Mol Biol Rev., 71(1):158-229 (2007)
15. Papadakos G, Wojdyla JA, Kleanthous C. Nuclease colicins and their immunity proteins. Q Rev Biophys., 45(1):57-103 (2012)
16. Esseku F, Adeyeye MC. Bacteria and pH-sensitive polysaccharide-polymer films for colon targeted delivery. Crit Rev Ther Drug Carrier Syst., 28(5):395-445 (2011)
17. Van den Mooter G. Colon drug delivery. Expert Opin Drug Deliv., 3(1):111-25 (2006)

## Claims

1. A therapeutic agent for use in a method of treating Crohn's disease, wherein the therapeutic agent is:
(i) a colicin;
(ii) a bacterium which produces and releases a colicin;
(iii) a food product comprising a bacterium which produces and releases a colicin; or
(iv) a polynucleotide that encodes a colicin.

2. A pharmaceutical composition for use in a method of treating Crohn's disease, comprising a pharmaceutically effective amount of a therapeutic agent which is:
(i) a colicin;
(ii) a bacterium which produces and releases a colicin; or
(iii) a polynucleotide that encodes a colicin;
and a pharmaceutically acceptable carrier.

3. The therapeutic agent or pharmaceutical composition according to claim 1 or claim 2, wherein the method comprises treatment of an adherent-invasive Escherichia coli (AIEC) infection.

4. The therapeutic agent or pharmaceutical composition according to any one of claims 1 to 3, wherein the method comprises treatment of an AIEC infection associated with a biofilm.

5. The therapeutic agent or pharmaceutical composition according to claim 3 or claim 4, wherein the method comprises treatment of an intracellular AIEC infection.

6. The therapeutic agent or pharmaceutical composition according to any one of the preceding claims, wherein the method of treating Crohn's disease is a prophylactic method of preventing Crohn's disease in a subject who has been determined to have a genetic predisposition to Crohn's disease.

7. A therapeutic agent for use in a method of treating an AIEC infection, wherein the therapeutic agent is:
(i) a colicin;
(ii) a bacterium which produces and releases a colicin;
(iii) a food product comprising a bacterium which produces and releases a colicin; or
(iv) a polynucleotide that encodes a colicin.

8. A pharmaceutical composition for use in a method of treating an AIEC infection, comprising a pharmaceutically effective amount of a therapeutic agent which is:
(i) a colicin;
(ii) a bacterium which produces and releases a colicin; or
(iii) a polynucleotide that encodes a colicin;
and a pharmaceutically acceptable carrier.

9. The therapeutic agent or pharmaceutical composition according to claim 7 or claim 8, wherein the AIEC of the infection are associated with a biofilm.

10. The therapeutic agent or pharmaceutical composition according to any one of claims 7 to 9, wherein the AIEC of the infection are intracellular.

11. The therapeutic agent or pharmaceutical composition according to claim 10, wherein the intracellular AIEC are in macrophages.

12. The therapeutic agent or pharmaceutical composition according to any one of the preceding claims, wherein the method is a method of treating a human.

13. The therapeutic agent or pharmaceutical composition according to any one of the preceding claims, wherein the therapeutic agent is a bacterium which produces and releases a colicin and which is an E. coli or Lactococcus lactis bacterium.

14. The therapeutic agent or pharmaceutical composition of any one of claims 1 to 12 wherein the therapeutic agent is a plasmid, which optionally further encodes an immunity protein and/or a colicin release protein.

15. The therapeutic agent or pharmaceutical composition according to any one of the preceding claims, wherein the colicin is a Group A colicin.

16. The therapeutic agent or pharmaceutical composition according to any one of the preceding claims, wherein the colicin:
(i) binds to an E.coli surface receptor selected from BtuB, FepA and Cir;
(ii) is a membrane-depolarising or pore-forming colicin, such as colicin E1 or colicin IA; or
(iii) is a nuclease, such as
(a) a DNase, e.g. colicin E9.
(b) an RNase, e.g. colicin E3
(c) a tRNase, e.g. colicin D.

17. An in vitro method of killing AIEC, the method comprising contacting the AIEC with an effective amount of a colicin or bacterium that produces a colicin.

## Patentansprüche

1. Therapeutikum zur Verwendung in einem Verfahren zur Behandlung von Morbus Crohn, wobei das Therapeutikum Folgendes ist:
(i) ein Colicin;
(ii) ein Bakterium, das ein Colicin produziert und freisetzt;
(iii) ein Nahrungsmittelprodukt, das ein Bakterium umfasst, das ein Colicin produziert und freisetzt; oder
(iv) ein Polynucleotid, das für ein Colicin kodiert.

2. Pharmazeutische Zusammensetzung zur Verwendung in einem Verfahren zur Behandlung von Morbus Crohn, umfassend eine pharmazeutisch wirksame Menge eines Therapeutikums, das Folgendes ist:
(i) ein Colicin;
(ii) ein Bakterium, das ein Colicin produziert und freisetzt; oder
(iii) ein Polynucleotid, das für ein Colicin kodiert;
und einen pharmazeutisch annehmbaren Träger.

3. Therapeutikum oder pharmazeutische Zusammensetzung nach Anspruch 1 oder Anspruch 2, wobei das Verfahren die Behandlung einer Infektion mit adhärentinvasivem *Escherichia coli* (AIEC) umfasst.

4. Therapeutikum oder pharmazeutische Zusammensetzung nach einem der Ansprüche 1 bis 3, wobei das Verfahren die Behandlung einer AIEC-Infektion umfasst, die mit einem Biofilm assoziiert ist.

5. Therapeutikum oder pharmazeutische Zusammensetzung nach Anspruch 3 oder Anspruch 4, wobei das Verfahren die Behandlung einer intrazellulären AIEC-Infektion umfasst.

6. Therapeutikum oder pharmazeutische Zusammensetzung nach einem der vorangegangenen Ansprüche, wobei das Verfahren zur Behandlung von Morbus Crohn ein prophylaktisches Verfahren zur Prävention von Morbus Crohn in einem Individuum ist, bei dem eine genetische Prädisposition für Morbus Crohn festgestellt wurde.

7. Therapeutikum zur Verwendung in einem Verfahren zur Behandlung einer AIEC-Infektion, wobei das Therapeutikum Folgendes ist:
(i) ein Colicin;
(ii) ein Bakterium, das ein Colicin produziert und freisetzt;
(iii) ein Nahrungsmittelprodukt, das ein Bakterium umfasst, das ein Colicin produziert und freisetzt; oder
(iv) ein Polynucleotid, das für ein Colicin kodiert.

8. Pharmazeutische Zusammensetzung zur Verwendung in einem Verfahren zur Behandlung einer AIEC-Infektion, umfassend eine pharmazeutisch wirksame Menge eines Therapeutikums, das Folgendes ist:
(i) ein Colicin;
(ii) ein Bakterium, das ein Colicin produziert und freisetzt; oder
(iii) ein Nahrungsmittelprodukt, das ein Bakterium umfasst, das ein Colicin produziert und freisetzt;
und einen pharmazeutisch annehmbaren Träger.

9. Therapeutikum oder pharmazeutische Zusammensetzung nach Anspruch 7 oder 8, wobei die AIEC der Infektion mit einem Biofilm assoziiert sind.

10. Therapeutikum oder pharmazeutische Zusammensetzung nach einem der Ansprüche 7 bis 9, wobei die AIEC der Infektion intrazellulär sind.

11. Therapeutikum oder pharmazeutische Zusammensetzung nach Anspruch 10, wobei die intrazellulären AIEC in Makrophagen vorliegen.

12. Therapeutikum oder pharmazeutische Zusammensetzung nach einem der vorangegangenen Ansprüche, wobei das Verfahren ein Verfahren zur Behandlung eines Menschen ist.

13. Therapeutikum oder pharmazeutische Zusammensetzung nach einem der vorangegangenen Ansprüche, wobei das Therapeutikum ein Bakterium ist, das ein Colicin produziert und freisetzt und das ein *E*.-*coli*- oder ein *Lactococcus-lactis-*Bakterium ist.

14. Therapeutikum oder pharmazeutische Zusammensetzung nach einem der Ansprüche 1 bis 12, wobei das Therapeutikum ein Plasmid ist, das gegebenenfalls weiters für ein Immunitätsprotein und/oder ein Colicin-Freisetzungsprotein kodiert.

15. Therapeutikum oder pharmazeutische Zusammensetzung nach einem der vorangegangenen Ansprüche, wobei das Colicin ein Gruppe-A-Colicin ist.

16. Therapeutikum oder pharmazeutische Zusammensetzung nach einem der vorangegangenen Ansprüche, wobei das Colicin:
(i) an einen *E*.-*coli*-Oberflächenrezeptor bindet, der aus BtuB, FepA und Cir ausgewählt ist;
(ii) ein membrandepolarisierendes oder porenbildendes Colicin wie etwa Colicin E1 oder Colicin IA ist; oder
(iii) eine Nuclease wie etwa
(a) eine DNase, z.B. Colicin E9,
(b) eine RNase, z.B. Colicin E3, oder
(c) eine tRNase, z.B. Colicin D,
ist.

17. In-vitro-Verfahren zum Abtöten von AIEC, wobei das Verfahren das Kontaktieren der AIEC mit einer wirksamen Menge eines Colicins oder eines Bakteriums, das ein Colicin produziert, umfasst.

## Revendications

1. Agent thérapeutique destiné à être utilisé dans un procédé de traitement de la maladie de Crohn, dans lequel l'agent thérapeutique est :
(i) une colicine ;
(ii) une bactérie qui produit et libère une colicine ;
(iii) un produit alimentaire comprenant une bactérie qui produit et libère une colicine ; ou
(iv) un polynucléotide qui code pour une colicine.

2. Composition pharmaceutique destinée à être utilisée dans un procédé de traitement de la maladie de Crohn, comprenant une quantité pharmaceutiquement efficace d'un agent thérapeutique qui est :
(i) une colicine ;
(ii) une bactérie qui produit et libère une colicine ;
(iii) un polynucléotide qui code pour une colicine ;
et un support pharmaceutiquement acceptable.

3. Agent thérapeutique ou composition pharmaceutique selon la revendication 1 ou la revendication 2, dans lequel le procédé comprend un traitement d'une infection par Escherichia Coli adhérentes-invasives (AIEC).

4. Agent thérapeutique ou composition pharmaceutique selon l'une quelconque des revendications 1 à 3, dans lequel le procédé comprend un traitement d'une infection AIEC associée à un biofilm.

5. Agent thérapeutique ou composition pharmaceutique selon la revendication 3 ou la revendication 4, dans lequel le procédé comprend un traitement d'une infection AIEC intracellulaire.

6. Agent thérapeutique ou composition pharmaceutique selon l'une quelconque des revendications précédentes, dans lequel le procédé de traitement de la maladie de Crohn est un procédé prophylactique de prévention de la maladie de Crohn chez un sujet qui a été déterminé comme ayant une prédisposition génétique à la maladie de Crohn.

7. Agent thérapeutique destiné à être utilisé dans un procédé de traitement d'une infection AIEC, dans lequel l'agent thérapeutique est :
(i) une colicine ;
(ii) une bactérie qui produit et libère une colicine ;
(iii) un produit alimentaire comprenant une bactérie qui produit et libère une colicine ; ou
(iv) un polynucléotide qui code pour une colicine.

8. Composition pharmaceutique destinée à être utilisée dans un procédé de traitement d'une infection AIEC, comprenant une quantité pharmaceutiquement efficace d'un agent thérapeutique qui est :
(i) une colicine ;
(ii) une bactérie qui produit et libère une colicine ;
(iii) un polynucléotide qui code pour une colicine ;
et un support pharmaceutiquement acceptable.

9. Agent thérapeutique ou composition pharmaceutique selon la revendication 7 ou la revendication 8, dans lequel les AIEC de l'infection sont associées à un biofilm.

10. Agent thérapeutique ou composition pharmaceutique selon l'une quelconque des revendications 7 à 9, dans lequel les AIEC de l'infection sont intracellulaires.

11. Agent thérapeutique ou composition pharmaceutique selon la revendication 10, dans lequel les AIEC intracellulaires sont dans des macrophages.

12. Agent thérapeutique ou composition pharmaceutique selon l'une quelconque des revendications précédentes, dans lequel le procédé est un procédé de traitement d'un être humain.

13. Agent thérapeutique ou composition pharmaceutique selon l'une quelconque des revendications précédentes, dans lequel l'agent thérapeutique est une bactérie qui produit et libère une colicine et qui est une bactérie E. coli ou Lactococcus lactis.

14. Agent thérapeutique ou composition pharmaceutique selon l'une quelconque des revendications 1 à 12, dans lequel l'agent thérapeutique est un plasmide, qui code en outre de manière facultative pour une protéine immunitaire et/ou une protéine libératrice de colicine.

15. Agent thérapeutique ou composition pharmaceutique selon l'une quelconque des revendications précédentes, dans lequel la colicine est une colicine de groupe A.

16. Agent thérapeutique ou composition pharmaceutique selon l'une quelconque des revendications précédentes, dans lequel la colicine :
(i) se lie à un récepteur de surface d'E. coli choisi parmi BtuB, FepA et Cir ;
(ii) est une colicine dépolarisatrice de membrane ou formatrice de pores, telle qu'une colicine E1 ou une colicine IA ; ou
(iii) est une nucléase, telle que
(a) une ADNase, par exemple colicine E9
(b) une ARNase, par exemple colicine E3
(c) un ARNase t, par exemple colicine D.

17. Un procédé in vitro pour tuer AIEC, le procédé comprenant la mise en contact des AIEC avec une quantité efficace d'une colicine ou d'une bactérie qui produit une colicine.
